# EUROPEAN PATENT SPECIFICATION

(11) **EP 2 962 718 B1**
(45) Date of publication and mention of the grant of the patent: **26.02.2020**
(21) Application number: 15172027.3
(22) Date of filing: 15.06.2015
(51) Int. Cl.: A61M 25/09, A61M 25/00

(54) **GUIDEWIRE**
FÜHRUNGSDRAHT
FIL-GUIDE

(30) Priority: 02.07.2014 JP 2014136428
(43) Date of publication of application: 06.01.2016
(73) Proprietor: Asahi Intecc Co., Ltd., Seto-shi, Aichi 489-0071 (JP)
(72) Inventor: Koike, Tadahiro c/o ASAHI INTECC CO., LTD., Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 2 415 497
- EP-A1- 2 417 998
- EP-A1- 2 514 475
- EP-B1- 1 324 799

## Description

### Field

The present invention relates to a guidewire for use as a medical device that is inserted into body cavities for purpose of treatment or examination.

### Background

Conventionally, various guidewires have been developed as a guide for a catheter or the like that is inserted into a tubular organ such as a blood vessel, a digestive tract, and a ureter or into body tissue for purpose of treatment or examination.

For example, JP H8-317989 A discloses a guidewire that includes a core wire, an outer coil provided about the outer circumference of a distal portion of the core wire, and an inner coil provided within the outer coil.

In using the guidewire disclosed in JP H8-317989 A in a lower limb region, for example, when a distal portion of the guidewire hits a hard lesion, the distal portion bends to an extreme degree and, if the bent guidewire is inserted deeper, the distal portion may bend to an even greater degree. As a result, plastic deformation is generated at the bended distal portion and plastic deformation makes it difficult to manually restore the shape of the distal portion. In other words, such a conventional guidewire has room for improvement in terms of suppression of excessive bending that occurs when the guidewire hits a hard lesion.

EP 1 324 799 B1 discloses a guidewire in accordance with the preamble of claim 1. Specifically, the guidewire comprises a shaft, an outer coil wound around a distal portion of the shaft, and an inner coil group provided within the outer coil and including four inner coils spaced apart from each other in a longitudinal direction of the shaft.

EP 2 514 475 A1 discloses a guidewire including a shaft, a multi-strand outer coil wound around a distal portion of the shaft, and an inner coil group provided within the outer coil and including a front-side coil portion and a proximal-side coil portion which are connected to each other.

EP 2 415 497 A1 discloses a guidewire including a shaft, and outer coil wound around a distal portion of the shaft, and a single inner coil provided within the outer coil.

Further, EP 2 417 998 A1 discloses a guidewire including a shaft, and outer coil wound around a distal portion of the shaft, and an inner coil group provided within the outer coil and including a front inner coil portion and a rear inner coil portion which are connected to each other.

Starting from EP 1 324 799 B1 an object of the present invention is to provide a guidewire which has an improved flexibility, an adequate restoring capacity when the guidewire hits a hard lesion and bends in a distal portion, an improved torque transmission and which ensures a reliable use.

### Solution to Problem

The above object is solved by a guidewire in accordance with the features of claim 1.

A guidewire of embodiment 1 of the present invention includes: a shaft having a distal end and a proximal end in a longitudinal direction,
an outer coil wound around a distal portion of the shaft, and
an inner coil group provided within the outer coil.

The inner coil group at least comprises a first inner coil provided within a distal portion of the outer coil and a second inner coil provided on a proximal side of the first inner coil,
a proximal end portion of the first inner coil is joined to the shaft via a first joining member, and a distal end portion of the second inner coil is joined to the shaft via a second joining member, and
the first joining member and the second joining member are spaced from each other in the longitudinal direction of the shaft.

The first inner coil and the second inner coil are wound around a distal portion of the shaft. The proximal end portion of the first inner coil comprises a proximal end of the first inner coil. The distal end portion of the second inner coil comprises a distal end of the second inner coil.

The materials to form the first joining member and the second joining member are not particularly limited. The examples of the materials include brazing metals such as Sn-Pb alloys, Pb-Ag alloys, Sn-Ag alloys, and Au-Sn alloys.

The proximal end of the first inner coil may project from the first joining portion toward the proximal end of the shaft, and the distal end of the second inner coil may project from the second joining portion toward the distal end of the shaft.

The outer diameter of the second inner coil may be substantially same as the outer diameter of the first inner coil, or the outer diameter of the second inner coil may be larger than the outer diameter of a first inner coil.

The outer coil may have an outer diameter increasing from the distal end toward the proximal end of the shaft.

Each of the first inner coil and the second inner coil is formed either by winding one wire (single-wire coil) or by winding a plurality of wires together (multi-wire coil). The wire forming the first inner coil or the second inner coil may be a single-strand wire consisting of a single strand or a multi-strand wire formed by twisting a plurality of strands together.

On the other hand, the outer coil is formed by winding a plurality of wires together (multi-wire coil). Each wire forming the outer coil is a multi-strand wire formed by twisting a plurality of strands together.

A guidewire of embodiment 2 of the present invention is the guidewire of embodiment 1 in which
the outer coil has an outer diameter that increases from the distal end toward the proximal end of the shaft, and
the second inner coil has a diameter greater than the diameter of the first inner coil.

A guidewire of embodiment 3 of the present invention is the guidewire of embodiment 1 or 2 in which
each of the first inner coil and the second inner coil is formed by winding a plurality of single-strand wires together (multi-wire coil).

### Advantageous Effects of Invention

In the guidewire of embodiment 1, the inner coil group includes the first inner coil and the second inner coil provided on the proximal side of the first inner coil, and the first joining member for joining the proximal end portion of the first inner coil and the second joining member for joining the distal end portion of the second inner coil are spaced from each other in the longitudinal direction.

Usually, when torque operation is applied to a handling portion of the guidewire and consequently winding of the outer coil is tightened, the outer coil deforms inwardly to reduce its diameter. When such deformation occurs to an excessive degree, a problem that a turns of the outer coil becomes displaced onto an adjacent turn occurs. In order to suppress such excessive deformation of the outer coil, the inner coil group is provided so as to support the outer coil. In this embodiment, the first inner coil and the second inner coil are provided as separate members and are spaced from each other. Therefore, the interaction between the first inner coil and the second inner coil is prevented when the outer coil deforms inwardly to get in touch with the inner coil.

When a distal portion of the guidewire hits a hard calcified lesion, for example, the guidewire bends in the distal portion. In this embodiment, the first joining member and the second joining member come into contact with each other and interfere with movement each other to suppress excessive bending and, as a result, plastic deformation of the distal portion of the guidewire is prevented. This allows the guidewire to ensure excellent reliability for consistent use even when the guidewire is used for insertion into the hard calcified lesion.

Further, the guidewire of this embodiment includes the outer coil that is formed by winding a plurality of multi-strand wires in a helical fashion. Since the strands forming the multi-strand wire are capable of slightly moving relative to each other, the outer coil has excellent flexibility, and an adequate restoring capacity. As a result, when the distal portion of the guidewire hits a hard calcified lesion and the guidewire bends in the distal portion, the shape of the guidewire can be manually restored with ease, which makes it possible to ensure a consistent use of the guidewire.

Furthermore, when torque is applied to the guidewire on the handling portion, not only the strands are pressed to each other but also the multi-strand wires are pressed to each other to increase contact pressure and enhance close contact. This improves torque transmission and ensures excellent passability of the guidewire.

In the guidewire of embodiment 2, the outer coil has an outer diameter that increases from the distal end toward the proximal end of the shaft, and the second inner coil has the diameter greater than the diameter of the first inner coil.

In other words, the first inner coil and the second inner coil conform to the shape of the outer coil having a diameter increasing from the distal end toward the proximal end, and the first inner coil and the second inner coil are spaced from each other by an appropriate distance to suppress inward deformation of the outer coil, and a proximal end of the first inner coil and a distal end of the second inner coil are spaced from each other in a direction perpendicular to the longitudinal direction, and, as a result, the interference between the proximal end of the first inner coil and the distal end of the second inner coil is reliably prevented.

This makes it possible, in the case of joining the distal end portion of the second inner coil after joining the proximal end portion of the first inner coil, for example, to efficiently perform a joining procedure with no interference occurring between the site of joining of the second inner coil (the distal end portion of the second inner coil) and the proximal end portion of the first inner coil.

The guidewire of embodiment 3 includes the inner coil that is formed by winding a plurality of single-strand wires together. This allows a joining material used for joining a certain site of the inner coil to preferentially penetrate into gaps between the wires along the longitudinal direction by capillary action, and, as a result, the resulting joining member (the first joining member and the second joining member) has an adequate volume and rigidity.

Therefore, when the distal portion of the guidewire, for example, hits the hard calcified lesion and the distal portion of the guidewire bends, the first joining member and the second joining member come into contact with each other and interfere with movement each other, and effectively suppress excessive bending and, as a result, plastic deformation of the distal portion of the guidewire is prevented even more reliably.

### Brief Description of Drawings

FIG. 1 is an expanded view of a partial cross-section of a guidewire according to a first reference embodiment.
FIG. 2 is an expanded view of a partial cross-section of a guidewire according to the first reference embodiment as it is bent.
FIG. 3 is an expanded view of a partial cross-section of a guidewire according to a second reference embodiment.
FIG. 4 is an expanded view of a partial cross-section of a guidewire according to a third reference embodiment.
FIG. 5 is an expanded view of a partial cross-section of a guidewire according to a fourth reference embodiment.
FIG. 6 is an expanded view of a partial cross-section of a guidewire according to a fifth reference embodiment.
FIG. 7 is an expanded view of a partial cross-section of a guidewire according to a sixth reference embodiment.
FIG. 8 is an expanded view of a partial cross-section of a guidewire according to a seventh reference embodiment.
FIG. 9 is a perspective view of an inner coil of a guidewire according to the seventh reference embodiment.
FIG. 10 is an expanded view of a partial cross-section of a guidewire according to an eighth reference embodiment.
FIG. 11 is a sectional view taken from line A-A of FIG. 10.
FIG. 12 is an expanded view of a partial cross-section of a guidewire according to an embodiment of the present invention.
FIG. 13 is a sectional view taken from line B-B of FIG. 12.

### Description of Embodiments

### [First reference embodiment]

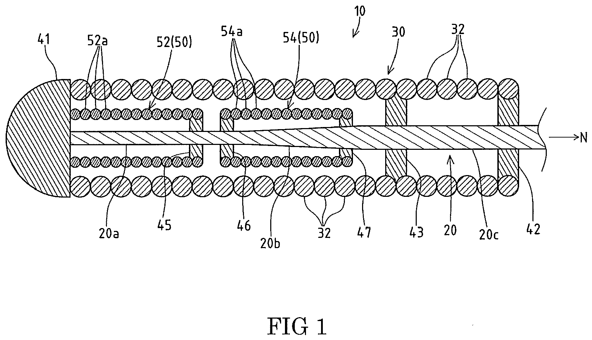

FIG. 1 is an expanded view of a partial cross-section of a guidewire according to a first reference embodiment. In FIG. 1, a distal side to be inserted into a body is shown to the left side, and a proximal side to be handled by a manipulator such as a doctor is shown to the right side.

A guidewire 10 shown in FIG. 1 is used, for example, in treating blood vessels of a lower limb by the Cross Over technique. The guidewire 10 includes a shaft 20, an outer coil 30 provided about the outer circumference of a distal portion of the shaft 20, and an inner coil group 50 provided within the outer coil 30.

The shaft 20 is explained first. The shaft 20 includes a smaller-diameter portion 20a, a tapered portion 20b, and a greater-diameter portion 20c, in this order from a distal end toward a proximal end. The smaller-diameter portion 20a is a portion of the shaft 20 at the most distal end and is the most flexible part of the shaft 20. The smaller-diameter portion 20a is formed flat by pressing. The tapered portion 20b is tapered with a circular cross section, with its diameter reduced toward the distal side. The greater-diameter portion 20c has a diameter greater than a diameter of the smaller-diameter portion 20a.

The material to form the shaft 20 is not particularly limited and includes a stainless steel (SUS304), a super-elastic alloy such as Ni-Ti alloys, piano wire, a cobalt-based alloy, or the like.

Next, the outer coil 30 is explained. The outer coil 30 of this reference embodiment is a single-wire coil formed by winding a single-strand wire 32 in a helical fashion. The outer diameter of the outer coil 30 is substantially uniform in the longitudinal direction N.

The material to form the outer coil 30 is not particularly limited and includes stainless steels such as martensitic stainless steel, ferritic stainless steel, austenitic stainless steel, austenitic-ferritic duplex stainless steel, and precipitation hardened stainless steel; super-elastic alloys such as Ni-Ti alloys; and metals radiopaque to X-rays, such as platinum, gold, tungsten, tantalum, iridium, and alloys thereof.

As shown in FIG. 1, the distal end of the outer coil 30 is fixed to the distal end of the shaft 20 via a distal joining member 41, the proximal end of the outer coil 30 is fixed to the shaft 20 via a proximal joining member 42, and a substantially middle portion that is located between the proximal joining member 42 and the distal joining member 41 is fixed to the shaft 20 via a middle joining member 43.

The materials to form the distal joining member 41, the proximal joining member 42, and the middle joining member 43 are not particularly limited and include brazing metals such as Sn-Pb alloys, Pb-Ag alloys, Sn-Ag alloys, and Au-Sn alloys.

Within the outer coil 30 to the distal side of the middle joining member 43, the inner coil group 50 is provided. The inner coil group 50 includes a first inner coil 52 provided within a distal portion of the outer coil 30 and a second inner coil 54 provided to the proximal side of the first inner coil 52.

In this reference embodiment, each of the first inner coil 52 and the second inner coil 54 is a single-wire coil formed by winding a single-strand wire 52a or 54a, respectively, in a helical fashion. An outer diameter of the first inner coil 52 is substantially the same as an outer diameter of the second inner coil 54.

The distal end of the first inner coil 52 is fixed to the distal end of the shaft 20 via the distal joining member 41, while the proximal end portion (the proximal end) of the first inner coil 52 is fixed to the shaft 20 via a first joining member 45.

The distal end portion (the distal end) of the second inner coil 54 is fixed to the shaft 20 via a second joining member 46, while the proximal end portion (the proximal end) of the second inner coil 54 is fixed to the shaft 20 via a third joining member 47.

The first joining member 45 for joining the proximal end portion of the first inner coil 52 and the second joining member 46 for joining the distal end portion of the second inner coil 54 are spaced from each other in the longitudinal direction N.

Usually, when torque operation is applied to the guidewire 10 on the handling portion of the guidewire and consequently winding of the outer coil 30 is tightened, the outer coil 30 deforms inwardly to reduce its diameter. When such deformation occurs to an excessive degree, a problem that a turn of the outer coil 30 becomes displaced onto an adjacent turn occurs. In order to suppress such excessive deformation of the outer coil, the inner coil is provided so as to support the outer coil. In this reference embodiment, the first inner coil and the second inner coil are provided as separate members and are spaced from each other, and an interaction between the first inner coil and the second inner coil is prevented when the outer coil deforms inwardly to come into contact with the inner coil.

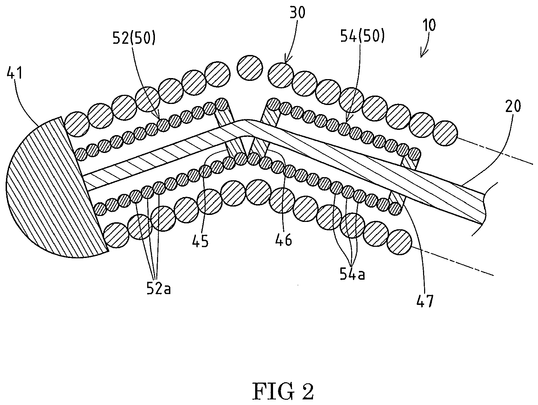

When the distal portion of the guidewire 10 hits a hard calcified lesion, for example, the guidewire 10 bends in the distal portion. In the guidewire 10 of this reference embodiment, as shown in FIG. 2, the first joining member 45 and the second joining member 46 come into contact with each other and interfere with movement each other to suppress excessive bending and, as a result, plastic deformation of the distal portion of the guidewire is prevented. This allows the guidewire 10 to ensure excellent reliability for consistent use even when the guidewire is used for insertion into the hard calcified lesion.

### [Second reference embodiment]

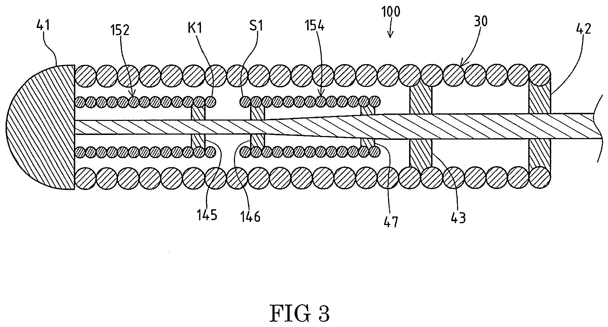

FIG. 3 is an expanded view of a partial cross-section of a guidewire according to a second reference embodiment. In FIG. 3, the distal side to be inserted into the body is shown to the left side, and the proximal side to be handled by a manipulator such as a doctor is shown to the right side. The same components as in the first reference embodiment are provided with the same reference numerals, with an overlapping explanation being omitted. The explanation below is mainly on differences.

In the first reference embodiment, the first joining member is provided for joining the proximal end of the first inner coil, and the second joining member is provided for joining the distal end of the second inner coil. In a guidewire 100 of the second reference embodiment, as shown in FIG. 3, a first joining member 145 is provided for joining a first inner coil 152 at a position that is located on the distal side of the proximal end K1 of the first inner coil, and a second joining member 146 is provided for joining a second inner coil 154 at a position that is located on the proximal side of the distal end S1 of the second inner coil. In other words, the proximal end K1 of the first inner coil 152 projects from the first joining member 145 toward the proximal end of the shaft 20 and the distal end S1 of the second inner coil 154 projects from the second joining member 146 toward the distal end of the shaft 20.

In the guidewire 100 of the second reference embodiment having such a configuration, as in the case of the first reference embodiment, when a distal portion of the guidewire 100 hits a hard calcified lesion, for example, the first joining member 145 and the second joining member 146 come into contact with each other and interfere with movement each other to suppress excessive bending and, as a result, plastic deformation of the distal portion of the guidewire is prevented.

### [Third reference embodiment]

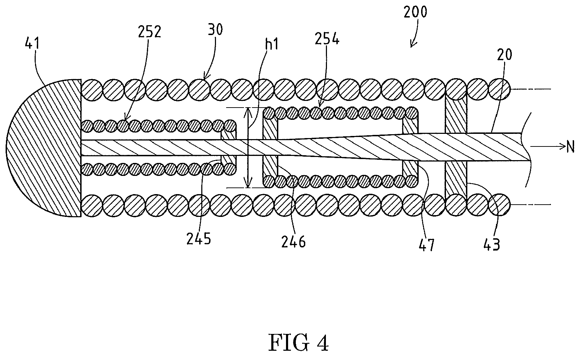

FIG. 4 is an expanded view of a partial cross-section of a guidewire according to a third reference embodiment. In FIG. 4, the distal side to be inserted into the body is shown to the left side, and the proximal side to be handled by a manipulator such as a doctor is shown to the right side. The same components as in the first reference embodiment are provided with the same reference numerals, with an overlapping explanation being omitted. The explanation below is mainly on differences.

In the first reference embodiment, the outer diameter of the first inner coil is substantially the same as the outer diameter of the second inner coil. On the other hand, in a guidewire 200 of the third reference embodiment, the outer diameter of a second inner coil 254 is larger than the outer diameter of a first inner coil 252. In addition, the proximal end portion of the first inner coil 252 is fixed to the shaft 20 via a first joining member 245, and the distal end portion of the second inner coil 254 is fixed to the shaft 20 via a second joining member 246.

In this reference embodiment, the second joining member 246 for joining the distal end portion of the second inner coil 254 has a width h1 (a height in the direction perpendicular to the longitudinal direction N) and a volume that are relatively greater than the width and the volume of the first joining member 245 for joining the proximal end portion of the first inner coil 252. As a result, when a distal portion of the guidewire 200 hits a hard calcified lesion and the guidewire 200 bends, for example, the second joining member 246 comes into contact with the first joining member 245 at a large area and consequently the first joining member 245 and the second joining member 246 surely come into contact with each other and interfere with movement each other, compared to the case in the first reference embodiment. As a result, the excessive bending of the guidewire 200 is suppressed and plastic deformation of the distal portion of the guidewire is effectively prevented.

In this reference embodiment, the outer diameter of the second inner coil 254 is larger than the outer diameter of the first inner coil 252 and a proximal end of the first inner coil 252 and a distal end of the second inner coil 254 are spaced from each other also in the direction perpendicular to the longitudinal direction N, and, as a result, the interference between the proximal end of the first inner coil and the distal end of the second inner coil is reliably prevented.

This makes it possible, in the case of joining the distal end portion of the second inner coil 254 after joining the proximal end portion of the first inner coil 252, for example, to efficiently perform a joining procedure with no interference occurring between the site of joining of the second inner coil 254 (the distal end portion of the second inner coil 254) and the proximal end portion of the first inner coil 252.

### [Fourth reference embodiment]

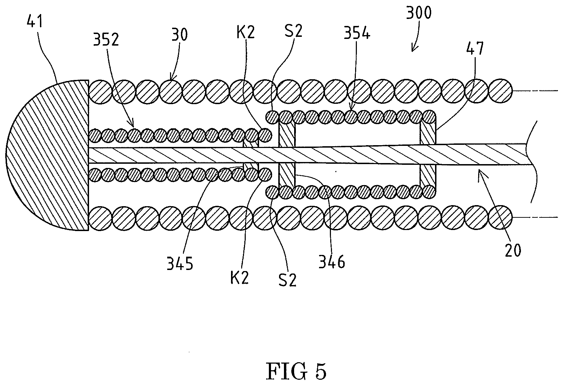

FIG. 5 is an expanded view of a partial cross-section of a guidewire according to a fourth reference embodiment. In FIG. 5, the distal side to be inserted into the body is shown to the left side, and the proximal side to be handled by a manipulator such as a doctor is shown to the right side. The same components as in the third reference embodiment are provided with the same reference numerals, with an overlapping explanation being omitted. The explanation below is mainly on differences.

In the third reference embodiment, the first joining member is provided for joining the proximal end of the first inner coil, and the second joining member is provided for joining the distal end of the second inner coil. On the other hand, in a guidewire 300 of the fourth reference embodiment, as shown in FIG. 5, a first joining member 345 is provided for joining a first inner coil 352 at a position that is located on the distal side of the proximal end K2 of the first inner coil 352, and a second joining member 346 is provided for joining a second inner coil 354 at a position that is located on the proximal side of the distal end S2 of the second inner coil 354. In other words, the proximal end K2 of the first inner coil 352 projects from the first joining member 345 toward the proximal end of the shaft 20 and the distal end S2 of the second inner coil 354 projects from the second joining member 346 toward the distal end of the shaft 20.

In the guidewire 300 of the fourth reference embodiment having such a configuration, as in the case of the third reference embodiment, when a distal portion of the guidewire 300 hits a hard calcified lesion, for example, the first joining member 345 and the second joining member 346 come into contact with each other and interfere with movement each other to suppress excessive bending and, as a result, plastic deformation of the distal portion of the guidewire is prevented.

### [Fifth reference embodiment]

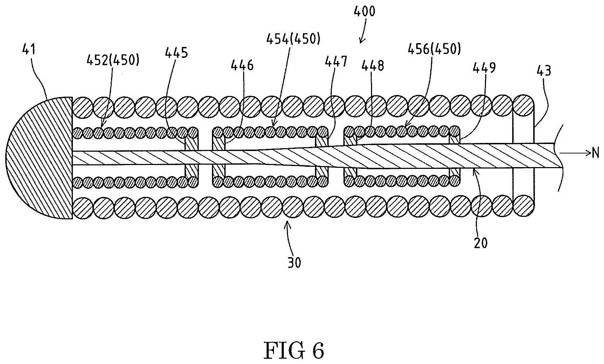

FIG. 6 is an expanded view of a partial cross-section of a guidewire according to a fifth reference embodiment. In FIG. 6, the distal side to be inserted into the body is shown to the left side, and the proximal side to be handled by a manipulator such as a doctor is shown to the right side. The same components as in the first reference embodiment are provided with the same reference numerals, with an overlapping explanation being omitted. The explanation below is mainly on differences.

In the first reference embodiment, the inner coil group includes two components, namely, the first inner coil and the second inner coil. On the other hand, in a guidewire 400 of this reference embodiment, an inner coil group 450 includes three components, namely, a first inner coil 452, a second inner coil 454, and a third inner coil 456, in this order from the distal side.

The proximal end portion of the first inner coil 452 is joined to the shaft 20 via a first joining member 445. The distal end portion of the second inner coil 454 is joined to the shaft 20 via a second joining member 446, and the proximal end portion of the second inner coil 454 is joined to the shaft 20 via a third joining member 447. The distal end portion of the third inner coil 456 is joined to the shaft 20 via a fourth joining member 448, and the proximal end portion of the third inner coil 456 is joined to the shaft 20 via a fifth joining member 449. The first joining member 445 for joining the proximal end portion of the first inner coil 452 and the second joining member 446 for joining the distal end portion of the second inner coil 454 are spaced from each other in the longitudinal direction N, and the third joining member 447 for joining the proximal end portion of the second inner coil 454 and the fourth joining member 448 for joining the distal end portion of the third inner coil 456 are spaced from each other in the longitudinal direction N.

When the distal portion of the guidewire 400 hits a hard calcified lesion and the guidewire 400 bends, for example, not only the interaction between the first joining member 445 and the second joining member 446 but also the interaction between the third joining member 447 and the fourth joining member 448 suppress excessive bending. As a result, plastic deformation of the distal portion of the guidewire is prevented more effectively.

Although the inner coil group of this reference embodiment includes three components (three inner coils), the number of components to form the inner coil group is not limited to three and may be four or greater.

### [Sixth reference embodiment]

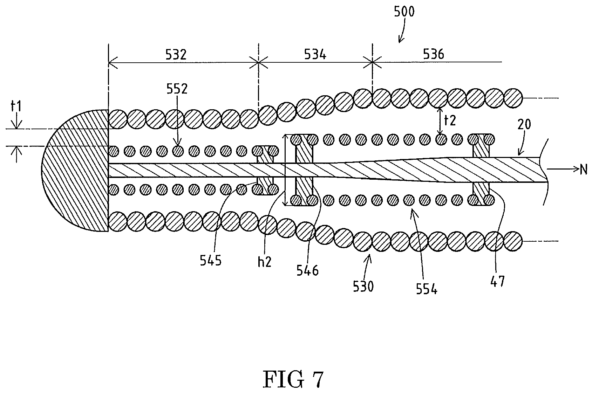

FIG. 7 is an expanded view of a partial cross-section of a guidewire according to a sixth reference embodiment. In FIG. 7, the distal side to be inserted into the body is shown to the left side, and the proximal side to be handled by a manipulator such as a doctor is shown to the right side. The same components as in the first reference embodiment are provided with the same reference numerals, with an overlapping explanation being omitted. The explanation below is mainly on differences.

In the first reference embodiment, the outer coil has an outer diameter that is uniform in the longitudinal direction. On the other hand, in a guidewire 500 of this reference embodiment, an outer coil 530 has an outer diameter that increases from the distal end toward the proximal end. In other words, the outer coil 530 of this reference embodiment includes a first linear portion 532, a tapered portion 534, and a second linear portion 536 that has an outer diameter larger than the outer diameter of the first linear portion 532, in this order from the distal side toward the proximal side.

This makes it possible, for example, to allow a distal portion (the first linear portion 532) of the guidewire 500 to be inserted into a narrowed lesion with ease and then to smoothly proceed deep inside by gradually pushing and expanding the narrowed lesion by the tapered portion 534.

In this reference embodiment, a second inner coil 554 has an outer diameter larger than the outer diameter of a first inner coil 552. A clearance t1 between the first inner coil 552 and the outer coil 530 (the first linear portion 532) is substantially the same as a clearance t2 between the second inner coil 554 and the outer coil 530 (the second linear portion 536). The clearance t1 (t2) is 5 to 10 µm, which is enough to suppress inward deformation of the outer coil 530 when the outer coil 530 is twisted in such a direction that the outer coil 530 is tightened.

In addition, in this reference embodiment where the first inner coil 552 and the second inner coil 554 conform to the shape of the outer coil 530 having a diameter increasing from the distal end toward the proximal end and are spaced from the outer coil 530 by the clearance t1 (t2) that is enough to suppress inward deformation of the outer coil 530, the proximal end of the first inner coil 552 and the distal end of the second inner coil 554 are spaced from each other also in the direction perpendicular to the longitudinal direction N, and, the interference between the proximal end of the first inner coil and the distal end of the second inner coil is reliably prevented.

This makes it possible, in the case of joining the distal end portion of the second inner coil 554 after joining the proximal end portion of the first inner coil 552, for example, to efficiently perform a joining procedure with no interference occurring between the site of joining of the second inner coil 554 (the distal end portion of the second inner coil 554) and the proximal end portion of the first inner coil 552.

In addition, in this reference embodiment, the second joining member 546 for joining the distal end portion of the second inner coil 554 has a width h2 (a height in the direction perpendicular to the longitudinal direction N) and a volume that are relatively greater than the width and the volume of the first joining member 545 for joining the proximal end portion of the first inner coil 552. As a result, when a distal portion of the guidewire 500 hits a hard calcified lesion and the guidewire 200 bends, for example, the second joining member 546 comes into contact with the first joining member 545 at a large area and consequently the first joining member 545 and the second joining member 546 surely come into contact with each other and interfere with movement each other, compared to the case in the first reference embodiment. As a result, the excessive bending of the guidewire 500 is suppressed and plastic deformation of the distal portion of the guidewire is effectively prevented.

### [Seventh reference embodiment]

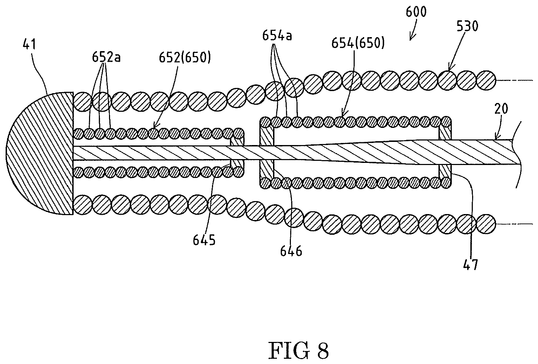

FIG. 8 is an expanded view of a partial cross-section of a guidewire according to a seventh reference embodiment. In FIG. 8, the distal side to be inserted into the body is shown to the left side, and the proximal side to be handled by a manipulator such as a doctor is shown to the right side. The same components as in the sixth reference embodiment are provided with the same reference numerals, with an overlapping explanation being omitted. The explanation below is mainly on differences.

In the sixth reference embodiment, the inner coil (the first inner coil and the second inner coil) is a single-wire coil formed by winding a single-strand wire in a helical fashion. On the other hand, in a guidewire 600 of this reference embodiment, each of a first inner coil 652 and a second inner coil 654 is formed by winding a plurality of single-strand wires.

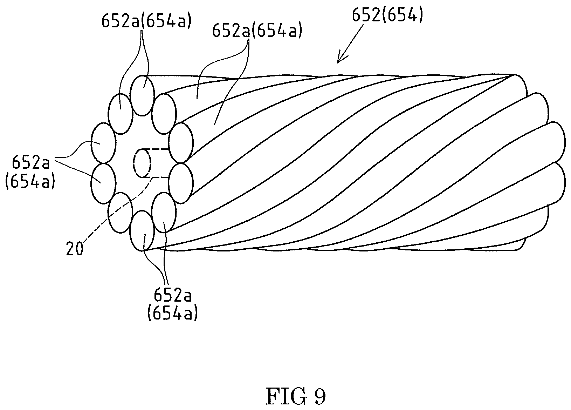

As shown in FIG. 9, in the guidewire of this reference embodiment includes a hollow inner coil (the first inner coil 652 and the second inner coil 654) that is formed by winding a plurality of single-strand wires (652a, 654a) (ten single-strand wires (652a, 654a) in this reference embodiment). This allows a joining material used for joining a certain site of the inner coil (652, 654) to preferentially penetrate into gaps between the single-strand wires (652a, 654a) along the longitudinal direction by capillary action, and, as a result, the resulting joining member (the first joining member 645 and the second joining member 646) has an adequate volume and rigidity.

Therefore, when the distal portion of the guidewire 600 hits a hard calcified lesion and the guidewire 600 bends, for example, the first joining member 645 and the second joining member 646 are come into contact with each other and surely interfere with movement each other to effectively suppress excessive bending. As a result, plastic deformation of the distal portion of the guidewire is prevented more reliably.

### [Eighth reference embodiment]

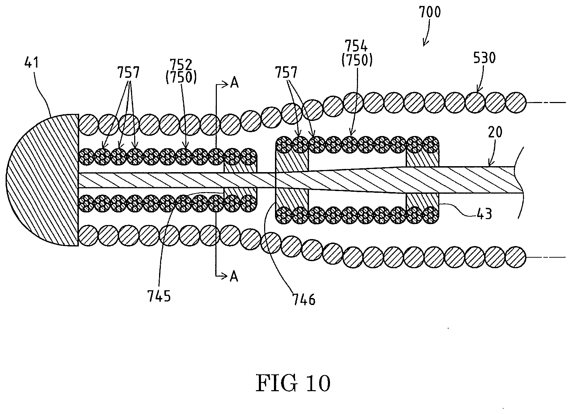

FIG. 10 is an expanded view of a partial cross-section of a guidewire according to an eighth reference embodiment. In FIG. 10, the distal side to be inserted into the body is shown to the left side, and the proximal side to be handled by a manipulator such as a doctor is shown to the right side. This drawing is merely a schematic view of the guidewire and the dimensional ratios including the cross-sectional profile of an inner coil formed of a plurality of multi-strand wires are different from the actual dimensional ratios. The same components as in the seventh reference embodiment are provided with the same reference numerals, with an overlapping explanation being omitted. The explanation below is mainly on differences.

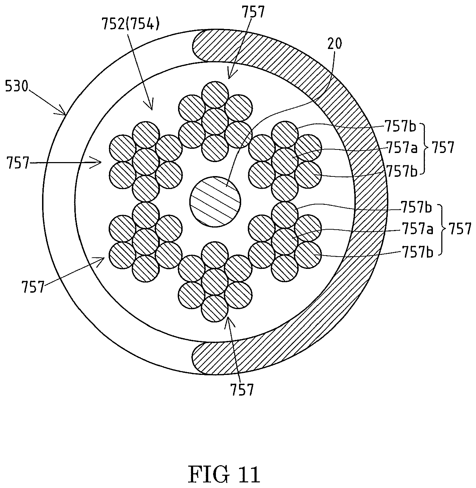

As shown in FIGS. 10 and 11, each of a first inner coil 752 and a second inner coil 754 of a guidewire 700 of this reference embodiment is formed by winding a plurality of multi-strand wires 757 in a helical fashion. More specifically, as shown in FIG. 11, the first inner coil 752 (the second inner coil 754) is formed by winding six multi-strand wires 757 in a helical fashion, each of the multi-strand wires 757 is formed by twisting a core strand 757a and six peripheral strands 757b wound around the outer circumference of the core strand 757a.

The materials to form the core strand 757a and the peripheral strands 757b in the first inner coil 752 (the second inner coil 754) are not particularly limited and include stainless steels such as martensitic stainless steel, ferritic stainless steel, austenitic stainless steel, austenitic-ferritic duplex stainless steel, and precipitation hardened stainless steel; super-elastic alloys such as Ni-Ti alloys; and metals radiopaque to X-rays, such as platinum, gold, tungsten, tantalum, iridium, and alloys thereof.

In the inner coil (752, 754) of this reference embodiment formed by winding a plurality of multi-strand wires 757 in a helical fashion, the multi-strand wires 757 are in close contact with each other and therefore gaps between the strands become small. As a result, in providing joining members 745 and 746 at certain sites of the inner coil (752, 754), penetration of the joining members (the joining members 745 and 746) into the gaps between the strands that constitute the inner coil (752, 754) along the longitudinal direction N is further facilitated and, as a result, the joining members 745 and 746 have large volumes and enhanced rigidity, compared to the case in the seventh reference embodiment.

Therefore, when the distal portion of the guidewire 700 hits a hard calcified lesion and the guidewire 700 bends, for example, the first joining member 745 and the second joining member 746 are come into contact with each other and even more surely interfere with movement each other to effectively suppress excessive bending. As a result, plastic deformation of the distal portion of the guidewire is prevented even more reliably.

### [Embodiment of the Present Invention]

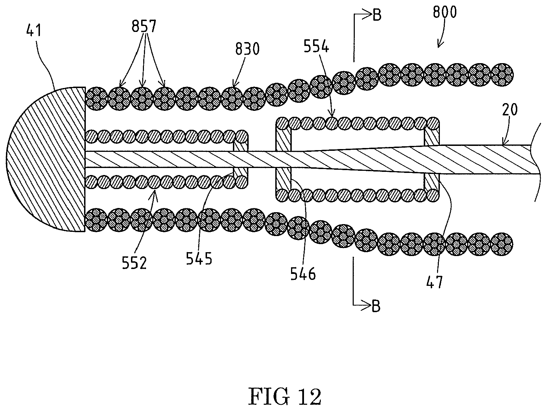

FIG. 12 is an expanded view of a partial cross-section of a guidewire according to an embodiment of the present invention. In FIG. 12, the distal side to be inserted into the body is shown to the left side, and the proximal side to be handled by a manipulator such as a doctor is shown to the right side. This drawing is merely a schematic view of the guidewire and the dimensional ratios including the cross-sectional profile of an outer coil formed of a plurality of multi-strand wires are different from the actual dimensional ratios. The same components as in the sixth reference embodiment are provided with the same reference numerals, with an overlapping explanation being omitted. The explanation below is mainly on differences.

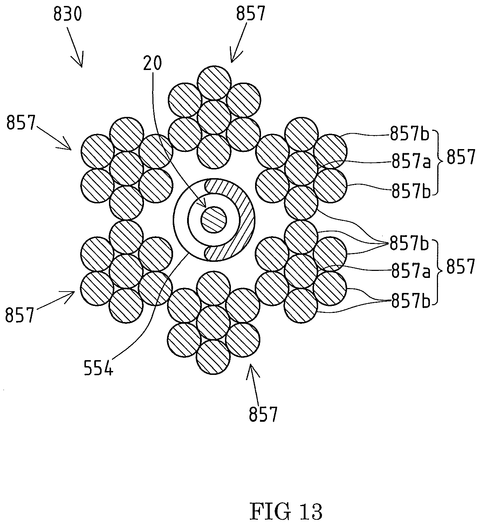

In the sixth reference embodiment, the outer coil is a single-wire coil formed by winding an single-strand wire in a helical fashion. On the other hand, as shown in FIG. 12, an outer coil 830 of a guidewire 800 of this embodiment is formed by winding a plurality of multi-strand wires 857 in a helical fashion. More specifically, as shown in FIG. 13, the outer coil 830 is formed by winding six multi-strand wires 857 in a helical fashion, each of the multi-strand wires 857 is formed by twisting a core strand 857a and six peripheral strands 857b wound around the outer circumference of the core strand 857a.

The materials to form the core strand 857a and the peripheral strand 857b in the outer coil 830 are not particularly limited and include stainless steels such as martensitic stainless steel, ferritic stainless steel, austenitic stainless steel, austenitic-ferritic duplex stainless steel, and precipitation hardened stainless steel; super-elastic alloys such as Ni-Ti alloys; and metals radiopaque to X-rays, such as platinum, gold, tungsten, tantalum, iridium, and alloys thereof.

The outer coil 830 of this embodiment is formed by winding a plurality of multi-strand wires 857 in a helical fashion. Since the strands 857a and 857b are capable of slightly moving relative to each other, the outer coil 830 has excellent flexibility, and an adequate restoring capacity. As a result, when a distal portion of the guidewire 800 hits a hard calcified lesion and the guidewire 800 bends in the distal portion, the shape of the guidewire 800 can be manually restored with ease, which makes it possible to ensure a consistent use of the guidewire 800.

Furthermore, when torque is applied to the guidewire 800 on a handling portion of the guidewire 800 to be handled, not only the strands are pressed to each other but also the multi-strand wires are pressed to each other to increase contact pressure and enhance close contact. This improves torque transmission and ensures excellent passability of the guidewire 800.

Although this embodiment employs the guidewire of the sixth reference embodiment as an example, the configuration of this embodiment may be applied to each of the other reference embodiments. Such a modification of the configuration does not create an adverse effect on function effect of the reference embodiments. Therefore, the modification makes it possible to ensure a consistent use of the guidewire, improve torque transmission, and ensure excellent passability.

### Reference Signs List

10, 100, 200, 300, 400, 500, 600, 700, 800: Guidewire
20: Shaft
30, 530, 830: Outer coil
50, 450, 650, 750: Inner coil group
52, 152, 252, 352, 452, 552, 652, 752: First inner coil
54, 154, 254, 354, 454, 554, 654, 754: Second inner coil
652a: Plurality of single-strand wires used in first inner coil
654a: Plurality of single-strand wires used second inner coil
757a, 757b: Plurality of strands of the multi-strand wires used in first inner coil and second inner coil
857: Multi-strand wire used in outer coil
N: Longitudinal direction

## Claims

1. A guidewire (800) comprising:
a shaft (20) having a distal end and a proximal end in a longitudinal direction,
an outer coil (830) wound around a distal portion of the shaft,
an inner coil group provided within the outer coil,
the inner coil group comprises at least a first inner coil (552) provided within a distal portion of the outer coil and a second inner coil (554) provided to a proximal side of the first inner coil,
a proximal end portion of the first inner coil (552) is joined to the shaft (20) via a first joining member (545), and a distal end portion of the second inner coil (554) is joined to the shaft (20) via a second joining member (546),
the first joining member (545) and the second joining member (546) are spaced from each other in the longitudinal direction of the shaft (20), and
the outer coil (830) is formed by winding at least one wire, **characterized in that**
the outer coil (830) is formed by winding a plurality of wires (857) in a helical fashion, and
each of the plurality of wires (857) forming the outer coil (830) is a multi-strand wire formed by twisting a plurality of strands (857a, 857b) together.

2. The guidewire according to claim 1, wherein
a proximal end (K1, K2) of the first inner coil projects from the first joining portion toward the proximal end of the shaft, and a distal end of the second inner coil projects from the second joining portion toward the distal end of the shaft.

3. The guidewire according to claim 1 or 2, wherein
the outer diameter of the second inner coil is substantially same as the outer diameter of the first inner coil.

4. The guidewire (800) according to claim 1 or 2, wherein
the outer diameter of the second inner coil (554754) is larger than the outer diameter of a first inner coil (552).

5. The guidewire (800) according to claim 4, wherein
the outer coil (830) has an outer diameter increasing from the distal end toward the proximal end of the shaft (20).

6. The guidewire (800) according to any one of claims 1 to 5, wherein
each of the first inner coil (552) and the second inner coil (554) is formed by winding at least one wire.

7. The guidewire (800) according to claim 6, wherein
the at least one wire is a single-strand wire consisting of a single strand.

8. The guidewire according to claim 6, wherein
the at least one wire is a multi-strand wire formed by twisting a plurality of strands together.

9. The guidewire according to any one of claims 1 to 8, wherein
each of the first inner coil and the second inner coil is formed by winding a plurality of wires together.

## Patentansprüche

1. Führungsdraht (800) mit:
einem Schaft (20), der in einer Längsrichtung ein distales Ende und ein proximales Ende hat,
einer äußeren Wicklung (830), die um einen distalen Abschnitt des Schafts gewickelt ist,
einer inneren Wicklungsgruppe, die innerhalb der äußeren Wicklung vorgesehen ist, wobei
die innere Wicklungsgruppe wenigstens eine erste innere Wicklung (552), die innerhalb eines distalen Abschnitts der äußeren Wicklung vorgesehen ist, und eine zweite innere Wicklung (554) umfasst, die auf einer proximalen Seite der ersten inneren Wicklung vorgesehen ist,
ein proximaler Endabschnitt der ersten inneren Wicklung (552) über ein erstes Fügeelement (545) an den Schaft (20) angefügt ist, und ein distaler Endabschnitt der zweiten inneren Wicklung (554) über ein zweites Fügeelement (546) an den Schaft (20) angefügt ist,
das erste Fügeelement (545) und das zweite Fügeelement (546) in der Längsrichtung des Schafts (20) voneinander beabstandet sind, und
die äußere Wicklung (830) aus einer Wicklung aus wenigstens einem Draht geformt ist, **dadurch gekennzeichnet, dass**
die äußere Wicklung (830) aus einer schraubenförmigen Wicklung aus einer Vielzahl von Drähten (857) geformt ist, und
jeder der Vielzahl von Drähten (857), die die äußere Wicklung (830) formen, ein aus einer Vielzahl von miteinander verdrillten Strängen (857a, 857b) geformter mehrsträngiger Draht ist.

2. Führungsdraht nach Anspruch 1, wobei
ein proximales Ende (K1, K2) der ersten inneren Wicklung über den ersten Fügeabschnitt hinaus in Richtung des proximalen Endes des Schafts ragt, und ein distales Ende der zweiten inneren Wicklung über den zweiten Fügeabschnitt hinaus in Richtung des distalen Endes des Schafts ragt.

3. Führungsdraht nach Anspruch 1 oder 2, wobei
der Außendurchmesser der zweiten inneren Wicklung im Wesentlichen gleich dem Außendurchmesser der ersten inneren Wicklung ist.

4. Führungsdraht (800) nach Anspruch 1 oder 2, wobei
der Außendurchmesser der zweiten inneren Wicklung (554754) größer ist als der Außendurchmesser der ersten inneren Wicklung (552).

5. Führungsdraht (800) nach Anspruch 4, wobei
die äußere Wicklung (830) einen von dem distalen Ende in Richtung des proximalen Endes des Schafts (20) zunehmenden Außendurchmesser hat.

6. Führungsdraht (800) nach einem der Ansprüche 1 bis 5, wobei
jede der ersten inneren Wicklung (552) und zweiten inneren Wicklung (554) aus einer Wicklung aus wenigstens einem Draht geformt ist.

7. Führungsdraht (800) nach Anspruch 6, wobei
der wenigstens eine Draht ein einsträngiger Draht bestehend aus einem einzelnen Strang ist.

8. Führungsdraht nach Anspruch 6, wobei
der wenigstens eine Draht ein mehrsträngiger Draht ist, der aus einer Vielzahl von miteinander verdrillten Strängen geformt ist.

9. Führungsdraht nach einem der Ansprüche 1 bis 8, wobei
jede der ersten inneren Wicklung und zweiten inneren Wicklung aus einer Vielzahl von miteinander verdrillten Drähten geformt ist.

## Revendications

1. Fil-guide (800) comprenant :
une tige (20) ayant une extrémité distale et une extrémité proximale dans une direction longitudinale,
une bobine externe (830) enroulée autour d'une partie distale de la tige,
un groupe de bobines internes prévu à l'intérieur de la bobine externe,
le groupe de bobines internes comprend au moins une première bobine interne (552) prévue dans une partie distale de la bobine externe et une seconde bobine interne (554) prévue sur un côté proximal de la première bobine interne,
une partie d'extrémité proximale de la première bobine interne (552) est assemblée à la tige (20) via un premier élément d'assemblage (545), et une partie d'extrémité distale de la seconde bobine interne (554) est assemblée à la tige (20) via un second élément d'assemblage (546),
le premier élément d'assemblage (545) et le second élément d'assemblage (546) sont espacés l'un de l'autre dans la direction longitudinale de la tige (20), et
la bobine externe (830) est formée en enroulant au moins un fil, **caractérisé en ce que**
la bobine externe (830) est formée en enroulant une pluralité de fils (857) d'une manière hélicoïdale, et
chacun de la pluralité de fils (857) formant la bobine externe (830) est un fil à plusieurs brins formés en torsadant une pluralité de brins (857a, 857b) ensemble.

2. Fil-guide selon la revendication 1, dans lequel :
une extrémité proximale (K1, K2) de la première bobine interne fait saillie de la première partie d'assemblage vers l'extrémité proximale de la tige, et une extrémité distale de la seconde bobine interne fait saillie de la seconde partie d'assemblage vers l'extrémité distale de la tige.

3. Fil-guide selon la revendication 1 ou 2, dans lequel :
le diamètre externe de la seconde bobine interne est sensiblement identique au diamètre externe de la première bobine interne.

4. Fil-guide (800) selon la revendication 1 ou 2, dans lequel
le diamètre externe de la seconde bobine interne (554754) est supérieur au diamètre externe d'une première bobine interne (552).

5. Fil-guide (800) selon la revendication 4, dans lequel
la bobine externe (830) a un diamètre externe augmentant de l'extrémité distale vers l'extrémité proximale de la tige (20).

6. Fil-guide (800) selon l'une quelconque des revendications 1 à 5, dans lequel
chacune parmi la première bobine interne (552) et la seconde bobine interne (554) est formée en enroulant au moins un fil.

7. Fil-guide (800) selon la revendication 6, dans lequel
le au moins un fil est un fil à brin unique se composant d'un seul brin.

8. Fil-guide selon la revendication 6, dans lequel
le au moins un fil est un fil à plusieurs brins formé en torsadant une pluralité de brins ensemble.

9. Fil-guide selon l'une quelconque des revendications 1 à 8, dans lequel
chacune parmi la première bobine interne et la seconde bobine interne est formée en enroulant une pluralité de fils ensemble.
